# EUROPÄISCHE PATENTSCHRIFT

(11) **EP 0 415 087 B1**
(45) Veröffentlichungstag und Bekanntmachung des Hinweises auf die Patenterteilung: **21.09.1994**
(21) Anmeldenummer: 90114431.1
(22) Anmeldetag: 27.07.1990
(51) Int. Cl.: A61F 13/20

(54) **Tampon für medizinische oder hygienische Zwecke sowie Verfahren zu seiner Herstellung**
Tampon for medical or hygienic purposes and its method of manufacture
Tampon à usage médical ou hygiénique et son procédé de fabrication

(30) Priorität: 30.08.1989 DE 3928677
(43) Veröffentlichungstag der Anmeldung: 06.03.1991
(73) Patentinhaber: VP - SCHICKEDANZ AG, D-90022 Nürnberg (DE)
(72) Erfinder: Kubicki, Jörn, Dr., D-8500 Nürnberg 30 (DE); Rink, Norbert, Dr., D-8508 Wendelstein 2 (DE)
(74) Vertreter: Rau, Manfred, Dr. Dipl.-Ing.

(56) Entgegenhaltungen:
- EP-A- 0 018 097
- EP-A- 0 360 180
- FR-A- 2 160 184
- GB-A- 2 203 949
- US-A- 3 428 044
- US-A- 3 784 425
- US-A- 3 821 350
- US-A- 4 027 673

## Beschreibung

Die Erfindung betrifft einen stäbchenförmigen mehrschichtigen Tampon für medizinische oder hygienische Zwecke nach dem Oberbegriff des Patentanspruches 1 sowie ein Verfahren zu seiner Herstellung.

Stäbchenförmige Tampons für hygienische Zwecke, insbesondere für Zwecke der Frauenhygiene, sind bekannt. Diese Tampons werden im allgemeinen aus einem spiralförmig aufgewickelten Wattezopf hergestellt, der durch radiales Verpressen verdichtet wird. Der Wattezopf besteht dabei in aller Regel aus einem Baumwoll-Zellwoll-Gemisch, dem gegebenenfalls noch Quellstoffe zur Erhöhung des Flüssigkeitsaufnahmevermögens hinzugefügt sind. An einem Ende ist das stäbchenförmige nach dem Pressen recht steife Produkt mit einem Rückhol faden ausgestattet, der dazu dient, den Tampon nach Gebrauch aus der Körperhöhle wieder zu entfernen.

Außer den erwähnten Hygiene-Tampons sind andere Tampon-Arten bekannt, die in erster Linie medizinischen Zwecken dienen. Sie werden ebenfalls in die Körperhöhle eingeführt, haben aber dort nicht den Hauptzweck Körperflüssigkeiten, beispielsweise Menstruationsflüssigkeiten, aufzusaugen, sondern Wirkstoffe abzugeben, die der Behandlung der Körperhöhle dienen. Ein derartiger Tampon ist beispielsweise in der deutschen Patentschrift 31 22 954 beschrieben. Es handelt sich um einen Tampon aus Polyvinylalkoholacetalschaumstoff, der mit Arzneimitteln ausgerüstet ist und in verschiedenen medizinischen Therapiebereichen angewendet werden kann.

Eines der Hauptprobleme bei Tampons, die die Bestimmung haben, Wirkstoffe an die Körperhöhle abzugeben, besteht darin, daß diese Wirkstoffe langsam und über einen möglichst großen Zeitraum abgegeben werden sollen, ohne daß die in umgekehrter Richtung verlaufende Flüssigkeitsaufnahme aus der Körperhöhle diese Wirkstoff-Freisetzung behindert. Es hat sich gezeigt, daß dieses Ziel mit Tampons aus Zellstoff und anderen Faserstoffen oder auch aus offenen Kunststoffschäumen, wie Polyurethan-, Polyvinylalkohol-, Cellulose- oder Gummischaum wegen ihrer Saugleistung nicht zufriedenstellend zu lösen ist.

Andererseits ist es bekannt, ringförmige Schwämmchen, welche mit kontrazeptiven Mitteln imprägniert sind, aus nativem Collagen in Form von faserförmigen Proteinen herzustellen und diese bei Bedarf in die Vaginalhöhle einzuführen. Schwammringe dieser Art sind beispielsweise in der US-PS 4 274 410 beschrieben. Das Material ist weich-elastisch, so daß es zur Herstellung von Tampons oder Medizinal-Tamponaden nicht geeignet ist. Es eignet sich aber gut als Trägerstoff für die erwähnten Wirkstoffe und hat sich insoweit bewährt.

Weiterhin ist aus der EP-A-0 018 097 ein Medizinaltampon bekannt, der einen Tamponkern sowie eine innere Membran und eine darauf laminierte äußere Membran aufweist. Die Wirkstoffe sind in der inneren Membran gelöst. Die charakteristischen Eigenschaften der Membranen sind so ausgelegt, daß bei der Verwendung des Tampons zumindest 58% der Wirkstoffe über einen bestimmten Zeitraum mit einer konstanten Rate freigesetzt werden, worauf nachfolgend die Freisetzungsrate expotentiell fällt. Aufgrund der für die Mebranen gewählten Materialien ist der Medizinaltampon nicht in der Lage Körperflüssigkeiten aufzunehmen.

Die US-A-34 28 044 zeigt einen Tampon, der auf seiner Außenseite mit einer Gleitmittelbeschichtung versehen ist. Letztere besteht aus Gelantine, die in Form eines Films auf den Tamponkern aufgebracht ist. Die Verwendung medizinischer Wirkstoffe ist bei diesem Tampon nicht vorgesehen.

Aus der US-A-37 84 425 ist ein Verfahren zur Herstellung von kunststoffummantelten Tampons aus organischem Fasermaterial bekannt. Dabei wird der Faserkern aus un- oder vorgepreßten Faserkörpern hergestellt und eine poröse Beschichtung aus wasserunlöslichem Kunststoff auf den Faserkörper aufgesprüht oder aufgesponnen. Faserkörper und Beschichtung werden anschließend zur Bildung des Tampons gepreßt.

Der Erfindung liegt die Aufgabe zugrunde, einen stäbchenförmigen selbsttragenden Tampon für medizinische oder hygienische Zwecke anzugeben, der auch ohne Zuhilfenahme eines Applikators eingeführt werden kann, der über längere Zeit als Wirkstoffdepot dienen kann und den Wirkstoff langsam mit etwa gleichbleibender Rate abgibt, ohne daß diese Abgabe durch die Flüssigkeitsaufnahme des Tampons behindert wird.

Zur Lösung dieser Aufgabe wird ein stäbchenförmiger mehrschichtiger Tampon mit den im Kennzeichnungsteil des Anspruches 1 angegebenen Merkmalen vorgeschlagen.

Der Wirkstoff wird dabei nicht in reiner Form im Tamponmantel deponiert, sondern gelöst in einem Retardstoff, beispielsweise einer geeigneten Fettmasse.

Durch den mehrschichtigen Aufbau wird erreicht, daß der Tampon hinreichend steif und selbsttragend gemacht werden kann, so daß er auch ohne Applikator in die Körperhöhle eingeführt werden kann. Daneben eröffnet die Anordnung eines Kernes aus gepreßtem Faserstoff die Möglichkeit der Anbringung eines Rückholfadens, so daß der Tampon nach längerem Gebrauch sicher aus der Körperhöhle entfernt werden kann. Ein solcher Rückholfaden wäre in einem Tampon, der aus Gelatine-Schaumstoff alleine besteht, nicht hinreichend fest zu verankern. Als besonders vorteilhaft hat es sich erwiesen, den Tamponkern aus gepreßten Cellulose- oder Celluloseacetatfasern herzustellen, da diese einen hinreichend festen Kern ergeben.

Der Tamponmantel weist im übrigen eine Dichte von 15 - 60 g/l sowie eine Porenanzahl von wenigstens 20 - 60 Poren/cm auf. Wie bereits gesagt, ist es vorteilhaft, wenn der im Tamponmantel enthaltene Wirkstoff in einem Retardstoff enthalten ist, mit dem der Tamponmantel dann imprägniert ist. Als Retardstoff eignen sich Tri- und/oder Partial-Glyceride höherer Fettsäuren mit einem Schmelzpunkt zwischen 34°C und 37°C. Der Wirkstoff kann vorteilhafterweise im Tamponmantel zur Erzielung eines erwünschten Freisetzungsverhaltens einen radial zu- oder abnehmenden Konzentrationsgradienten bilden. Eine solche Anordnung des Wirkstoffes im Mantel kann erzielt werden, wenn der Mantel je nach Bedarf nach seiner Herstellung von innen oder von außen mit Wirkstoff bzw. einer Wirkstofflösung in Retardstoff imprägniert wird.

Der Tamponmantel soll vorzugsweise aus luft- oder gefriergetrocknetem Collagen- oder Gelatine-Schaumstoff bestehen, der mit Hilfe von aldehydischen Vernetzungsmitteln gehärtet oder nachgehärtet ist. Das Vernetzungsmittel kann schon bei der Herstellung des Schaumstoffes in diesen eingegeben werden. Andererseits ist es auch möglich, das Vernetzungsmittel erst später mit dem Schaumstoff in Berührung zu bringen, wenn der Schaumstoff schon zu einem Tamponmantel verarbeitet ist. Als Vernetzungsmittel kommen gasförmige oder flüssige Aldehyde, wie beispielsweise Formaldehyd, Glutaraldehyd oder ähnliche Stoffe in Betracht.

Wichtig ist, daß der Tamponkern und der Tamponmantel hinreichend fest miteinander verbunden sind. Beim Herstellungsverfahren nach Anspruch 9 kann es dabei vorteilhaft sein, die beiden Teile miteinander zu verkleben, beispielsweise mit Hilfe einer Gelatine- oder einer Collagen-Haftschicht, die auf den Tamponkern aufgetragen wird.

Der Rückholfaden sollte eine Schlaufe bilden, die den Tamponkern und den Tamponmantel durchdringt. Auf diese Weise wird verhindert, daß sich die beiden Teile beim Entfernen des Tampons aus der Körperhöhle voneinander lösen.

Schließlich wird vorgeschlagen, die Oberfläche des Tampons mit einer Gleitmittelbeschichtung zu versehen, wie dies grundsätzlich bekannt ist. Hierzu soll ein Hartfett verwendet werden, welches bei etwa 34,5°C, also bei Körpertemperatur, erweicht.

Bei Versuchen hat es sich als vorteilhaft erwiesen, wenn der Durchmesser des Tamponkerns etwa ein Drittel des Gesamtdurchmessers des Tampons beträgt. Tolerierbar ist aber auch ein größerer Bereich von etwa einem Viertel bis drei Viertel des Gesamtdurchmessers.

Die Erfindung ist im folgenden anhand der beiliegenden Zeichnung näher erläutert.

Es stellen dar:
Fig. 1 bis 3 verschiedene Ausführungsformen des Tampons;
Fig. 4 ein Schaubild.

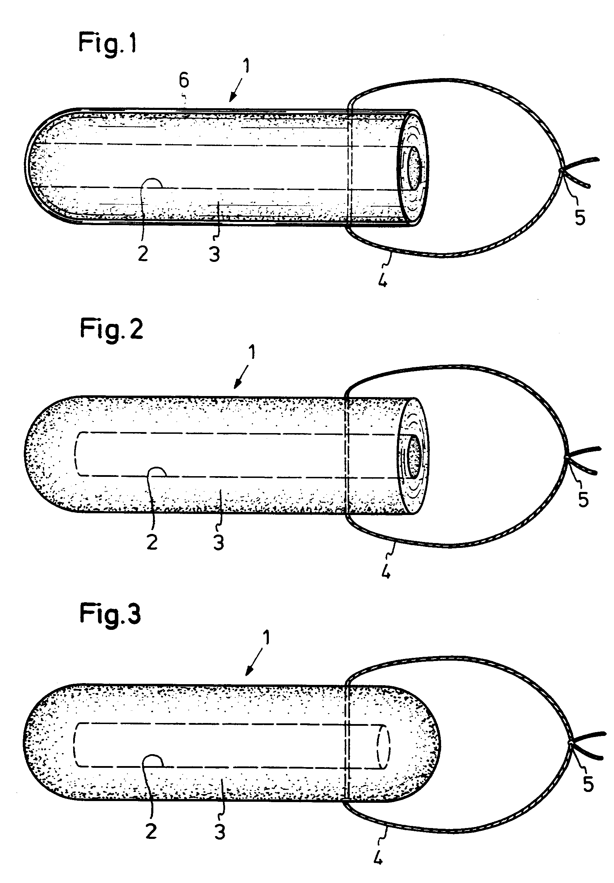

In der Zeichnung ist der Tampon als Ganzes jeweils mit 1 bezeichnet. Die Zeichnung läßt erkennen, daß der Tampon aus einem Tamponkern 2 und einem Tamponmantel 3 besteht. Der Tamponkern besteht aus gepreßtem Faserstoff, vorzugsweise aus gepreßten Celluloseacetatfasern. Der Tamponmantel besteht aus nachgehärtetem Collagen- oder Gelatine-Schaumstoff, der mit einer wirkstoffhaltigen Fettstoffmasse imprägniert ist. Beim Ausführungsbeispiel gemäß Fig. 1 handelt es sich um einen Tampon mit praktisch durchgehendem Tamponkern 2. Beim Ausführungsbeispiel gemäß Fig. 2 ist der Tamponkern 2 von hinten eingeführt und nimmt nur etwa drei Viertel der Tamponlänge ein. Beim Ausführungsbeispiel gemäß Fig. 3 liegt der Tamponkern 2 im Innern des Tampons und ist allseitig von Schaumstoff umschlossen.

Die Art des Wirkstoffes richtet sich nach der Art der vorzunehmenden Behandlung; es kann sich um Antibiotika, Sulfonamide, Antimykotika, Hormone oder auch andere Wirkstoffe handeln.

Wie die Zeichnung zeigt, sind die beiden Bestandteile des Tampons, also Kern und Mantel, von einem Rückholfaden 4 durchsetzt, der an seinem Ende über einen Knoten 5 zu einer Schlaufe geschlossen ist. Die Oberfläche des Tamponmantels ist vorzugsweise noch mit einer Gleitmittelhaut 6 überzogen.

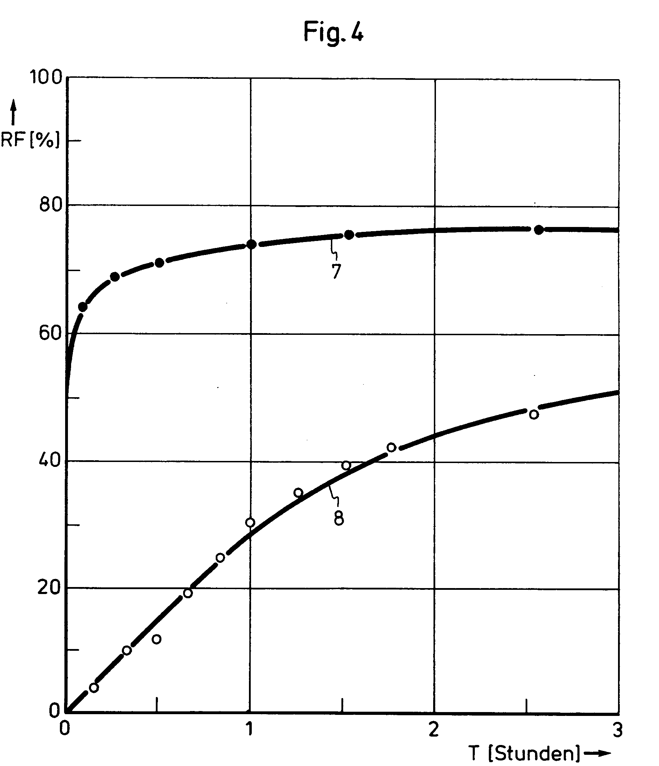

Das Diagramm gemäß Fig. 4 zeigt einen Vergleich der relativen Freisetzung RF einer Suppositorienmasse aus zwei unterschiedlich aufgebauten Tampons in einer Laborapparatur zur Bestimmung der Wirkstoff-Freisetzung. Der Tampon befindet sich hierzu in einem Dialyseschlauch, der von außen einem hydrostatischen Druck von 35 cm Wassersäule ausgesetzt ist. Durch das Innere des Dialyseschlauches wird physiologische Kochsalzlösung mit einer Fließgeschwindigkeit von 0,2 ml/10 Minuten geleitet. Die Versuchstemperatur betrug 37°C. Die Kurven zeigen die zeitliche Freisetzung der Suppositorienmasse aus den beiden Tampons in Prozent.

Bei der oberen Kurve 7 handelt es sich um einen Zellstoffkern von 0,8 cm Durchmesser und 5 cm Länge, der äußerlich mit 4 g Suppositorienmasse beschichtet war. Dieser Kern repräsentiert einen Tampon der nicht retardierten Form.

Die untere Kurve 8 zeigt das Verhalten eines gleich großen Gelatine-Tampons, dessen Gelatine-Schaumstoffmantel mit 4 g der gleichen Suppositorienmasse imprägniert ist. Es ist erkennbar, daß die Rate der relativen Freisetzung bei diesem Tampon (Kurve 8) weitaus langsamer und gleichmäßiger ansteigt als di es beim nicht retardierten Tampon (Kurve 7) der Fall ist. In gleicher Weise würde dann auch der in der Suppositorienmasse gelöste Wirkstoff freigesetzt und an das Innere der Körperhöhle abgegeben.

Der vorgeschlagene Mehrschichten-Tampon kann auf verschiedene Weise hergestellt werden.

Bei einem bevorzugten Verfahren wird so vorgegangen, daß zunächst ein Tamponkern durch Pressen von Faserstoff, beispielsweise Zellstoff, Celluloseacetatfasern oder dergl. zu einem Stäbchen hergestellt wird. In einem weiteren Arbeitsschritt wird ein Tamponmantel aus Collagen- oder Gelatine-Schaumstoff gefertigt, wobei in den Mantel eine zentrische Längsöffnung eingearbeitet wird, deren Innendurchmesser dem Außendurchmesser des zylindrischen Tamponkerns entspricht. Je nach Zusammensetzung und Art der Collagen- bzw. Gelatinemasse ist es erforderlich, den Schaumstoff zu härten. Es geschieht dies in bekannter Weise durch geeignete Aldehyde, beispielsweise Formaldehyd, Glutaraldehyd oder dergl. Das Härtereagenz kann dabei dem Schaumstoff unmittelbar zugegeben oder auch nachher in den Schaumstoff eingebracht werden. In einem weiteren Arbeitsschritt wird der Tamponkern mit einer Collagen- oder Gelatine-Haftschicht umhüllt und sodann der Kern in die Längsöffnung des Tamponmantels eingeführt. Schließlich wird ein Rückholfaden durch den Tamponkern sowie den Tamponmantel gezogen und die Enden des Rückholfadens werden miteinander verknotet. Danach wird der Tampon durch Tränken oder Besprühen mit der den Wirkstoff enthaltenden auf 40 - 50°C temperierten Fettschmelze imprägniert. Anschließend läßt man den Tampon am Umfang auf unter 20°C abkühlen. Dann wird der Tampon im Tauch- oder Sprühverfahren mit der etwa 40°C warmen Gleitmittelschmelze überzogen.

Ein anderes ebenfalls bevorzugtes Herstellungsverfahren ist durch folgende Arbeitsschritte gekennzeichnet:

Zunächst wird wiederum ein Tamponkern durch Pressen von Faserstoff zu einem Stäbchen hergestellt. Als nächstes wird ein Rückholfaden durch den Tamponkern gezogen und der Faden durch Verknoten der Enden miteinander zu einer Schlaufe geformt. Der Tamponkern wird alsdann zentrisch in eine Form eingesetzt, die entweder zuvor oder nachträglich mit Collagen- oder Gelatine-Schaum, der aus einem Extruder austritt, ausgespritzt wird. Bei diesem Verfahren verbinden sich Kern und Schaumstoffmantel fest miteinander, so daß ein gesondertes Umhüllen des Kernes mit einer Gelatine-Haftschicht nicht erforderlich ist. Nach Fertigstellen des rohen Tampons wird dieser durch Tränken oder Besprühen mit einer den Wirkstoff enthaltenden Fettstoffschmelze imprägniert. Danach kann gegebenenfalls zusätzlich eine Gleitmittelbeschichtung im Tauch- oder Sprühverfahren aufgebracht werden.

Als Imprägnierungs- oder Beschichtungsmasse kommen u. a. folgende Stoffe oder Stoffgemische in Frage:
- Tri- oder Partialglyceride von Fettsäuren mit 12 - 18 C-Atomen;
- Polyethylenglykol;
- Polyethylen-Sorbitan-Fettsäureester;
- Cetylphthalat;
- Propylenglykol-Mono- und -Distearat;
- verzweigte Fettalkohole.

Die genannten Stoffe können auch in Mischung miteinander verwendet werden.

### Beugszeichenliste:

- 1 =: Tampon
- 2 =: Tamponkern
- 3 =: Tamponmantel
- 4 =: Rückholfaden
- 5 =: Knoten
- 6 =: Gleitmittel haut
- 7 =: obere Kurve
- 8 =: untere Kurve

## Patentansprüche

1. Stäbchenförmiger mehrschichtiger Tampon für medizinische oder hygienische Zwecke, mit folgenden Merkmalen:
- einem Tamponkern (2) aus gepreßtem Faserstoff;
- einem Rückholfaden (4);
- gegebenenfalls einer äußeren Gleitmittelbeschichtung (6);
gekennzeichnet durch
- einen Tamponmantel (3) aus gehärtetem Collagen- oder Gelatine-Schaum, der mit Wirkstoffen imprägniert ist, wobei
der Tamponmantel (3) eine Dichte von 15 - 60 g/l sowie eine Porenzahl von wenigstens 20 - 60 Poren/cm aufweist und
mit einem Retardstoff imprägniert ist, in welchem der Wirkstoff gelöst ist.

2. Tampon nach Anspruch 1,
dadurch gekennzeichnet,
daß der Tamponkern aus gepreßten Zellstoff-, Baumwoll- oder Celluloseacetat-Fasern besteht.

3. Tampon nach Anspruch 1 oder 2,
dadurch gekennzeichnet,
daß der Tamponkern (2) und der Tamponmantel (3) fest miteinander verbunden, insbesondere miteinander verklebt sind.

4. Tampon nach einem der Ansprüch 1-3,
dadurch gekennzeichnet,
daß der Retardstoff aus Tri- und/oder Partial-Glyceriden höherer Fettsäuren mit einem Schmelzpunkt zwischen 34°C und 37°C besteht.

5. Tampon nach einem der vorausgehenden Ansprüche,
dadurch gekennzeichnet,
daß der Wirkstoff im Tamponmantel zur Erzielung eines erwünschten Freisetzungsverhaltens einen radial zu- oder abnehmenden Konzentrationsgradienten bildet.

6. Tampon nach einem der vorausgehenden Ansprüche,
dadurch gekennzeichnet,
daß der Rückholfaden (4) eine Schlaufe bildet, die den Tamponkern und den Tamponmantel durchdringt.

7. Tampon nach einem der vorausgehenden Ansprüche,
dadurch gekennzeichnet,
daß die Gleitmittelbeschichtung aus einem bei etwa 30 - 35°C, vorzugsweise bei 34,5°C erweichenden Hartfett besteht.

8. Tampon nach einem der vorausgehenden Ansprüche,
dadurch gekennzeichnet,
daß der Durchmesser des Tamponkernes etwa ein Drittel des Gesamtdurchmessers des Tampons beträgt.

9. Verfahren zum Herstellen eines Tampons nach einem der Ansprüche 1-8,
gekennzeichnet durch folgende Arbeitsschritte:
- Herstellung eines Tamponkernes (2) durch Pressen von Faserstoff zu einem Stäbchen;
- Herstellen eines Tamponmantels (3) aus Collagen- oder Gelatine-Schaumstoff mit zentrisch eingearbeiteter Längsöffnung, deren Innendurchmesser dem Außendurchmesser des zylindrischen Tamponkernes entspricht;
- Umhüllung des Tamponkernes (2) mit einer Collagen- oder Gelatine-Haftschicht;
- Einsetzen des beschichteten Tamponkernes (2) in die Längsöffnung des Tamponmantels (3);
- Durchziehen des Rückholfadens (4) durch den Tamponkern (2) und Tamponmantel (3) nahe dem Tamponende und Bilden einer Schlaufe durch Verknoten der Enden des Rückholfadens;
- Imprägnieren des Tampons durch Tränken oder Besprühen mit der den Wirkstoff enthaltenden auf 40 - 50°C temperierten Fettschmelze;
- Abkühlung am Umfang auf unter 20°C;
- Überziehen des Tampons mit der etwa 40°C warmen Gleitmittelschmelze im Tauch- oder Sprühverfahren.

## Claims

1. Uvular multi-layer tampon for medical or hygienic use, comprising the following features:
- a tampon core (2) of a compressed fibrous material;
- a withdrawal cord (4);
- possibly, an external slip additive film (6);
characterized by
- a tampon coating (3) of a cured collagen or gelatin foam impregnated with drugs,
the tampon coating (3) having a density of 15 - 60 g/l as well as a pore count of at least 20 - 60 pores/cm, and
being impregnated with a retarding agent in which the drug is dissolved.

2. Tampon according to claim 1,
characterized in that
the tampon core consists of compressed cellulose, cotton or cellulose acetate fibers.

3. Tampon according to claim 1 or 2,
characterized in that
the tampon core (2) and the tampon coating (3) are tightly joined, in particular glued together.

4. Tampon according to one of the claims 1 to 3,
characterized in that
the retarding agent consists of triglycerides and/or partial glycerides of higher fatty acids with a melting point of between 34°C and 37°C.

5. Tampon according to one of the preceding claims,
characterized in that
for a desired release behavior to be achieved, the drug in the tampon coating forms a radially increasing or decreasing gradient of concentration.

6. Tampon according to one of the preceding claims,
characterized in that
the withdrawal cord (4) forms a loop that passes through the tampon core and the tampon coating.

7. Tampon according to one of the preceding claims,
characterized in that
the slip additive film consists of a hard fat softening at about 30 - 35°C, preferably at 34,5°C.

8. Tampon according to one of the preceding claims,
characterized in that
the diameter of the tampon core is about one third of the total diameter of the tampon.

9. Method of manufacture of a tampon according to one of the claims 1 - 8,
characterized by the following steps:
- manufacturing a tampon core (2) by compressing a fibrous material to form a uvular body;
- manufacturing a tampon coating (3) from a collagen or gelatin foam with a centrally integrated longitudinal opening of which the internal diameter corresponds to the external diameter of the cylindrical tampon core;
- covering the tampon core (2) with a collagen or gelatin adhesive layer;
- inserting the covered tampon core (2) into the longitudinal opening of the tampon coating (3);
- passing the withdrawal cord (4) through the tampon core (2) and the tampon coating (3) in the proximity of the tampon end and forming a loop by knotting the ends of the withdrawal cord together;
- impregnating the tampon by soaking or spraying with the melt of fatty matters heated to 40 - 50°C and containing the drug;
- cooling below 20°C on the circumference;
- coating the tampon with the slip additive melt of a temperature of about 40°C in the dipping or spraying method.

## Revendications

1. Tampon multicouches en forme de bâtonnet pour usage médical ou hygiénique, ayant les particularités ci-après :
- un noyau (2) de tampon en matière fibreuse comprimée ;
- un cordonnet de retrait (4) ;
- le cas échéant, un revêtement extérieur (6) avec un agent antifriction ; caractérisé par
- une enveloppe (3) de tampon en mousse de collagène ou de gélatine durcie imprégnée de matières actives,
l'enveloppe (3) du tampon ayant un poids sépcifique de 15 à 60 g/l ainsi qu'une porosité d'au moins 20 à 60 pores/cm, et
étant imprégnée d'une substance de retardement dans laquelle la matière active est dissoute.

2. Tampon selon la revendication 1, caractérisé en ce que le noyau du tampon est composé de libres de cellulose, de coton ou d'acétate de cellulose.

3. Tampon selon l'une des revendications 1 ou 2, caractérisé en ce que le noyau (2) du tampon et l'enveloppe (3) du tampon sont solidement reliés entre eux, en particulier collés entre eux.

4. Tampon selon l'une quelconque des revendications 1 à 3, caractérisé en ce que la substance de retardement est composée de tri- et/ou de glycérides partielles d'acides gras supérieurs ayant un point de fusion entre 34 °C et 37 °C.

5. Tampon selon l'une quelconque des revendications précédentes, caractérisé en ce que, pour obtenir un comportement souhaité de libération, la matière active forme dans l'enveloppe du tampon un gradient de concentration croissant ou décroissant radialement.

6. Tampon selon l'une quelconque des revendications précédentes, caractérisé en ce que le cordonnet de retrait (4) forme une boucle qui traverse le noyau du tampon et l'enveloppe du tampon.

7. Tampon selon l'une quelconque des revendications précédentes, caractérisé en ce que le revêtement antifriction est composé d'une graisse dure se ramollissant à environ 30 à 35 °C, de préférence à 34,5 °C.

8. Tampon selon l'une quelconque des revendications précédentes, caractérisé en ce que le diamètre du noyau du tampon représente environ un tiers du diamètre de l'ensemble du tampon.

9. Procédé destiné à la fabrication d'un tampon selon l'une quelconque des revendications 1 à 8, caractérisé par les phases opératoires ci-après :
- fabrication d'un noyau (2) de tampon par compression d'une matière fibreuse en un bâtonnet ;
- fabrication d'une enveloppe (3) de tampon en mousse de collagène ou de gélatine comportant une ouverture longitudinale pratiquée centralement, dont le diamètre intérieur correspond au diamètre extérieur du noyau cylindrique du tampon ;
- enrobage du noyau (2) du tampon par une couche d'adhérence en collagène ou en gélatine ;
- insertion du noyau (2) du tampon dans l'ouverture longitudinale de l'enveloppe (3) du tampon ;
- mise en place du cordonnet de retrait (4) en le faisant passer à travers le noyau (2) du tampon et l'enveloppe (3) du tampon à proximité de l'extrémité du tampon et formation d'une boucle en attachant les extrémités du cordonnet de retrait ;
- imprégnation du tampon par imbibation ou pulvérisation de la graisse fondue contenant lamatière active, portée à une température de l'ordre de 40 à 50 °C ;
- refroidissement en périphérie à une température inférieure à 20° C ;
- revêtement du tampon avec l'agent antifriction fondu porté à une température d'environ 40 °C, par un procédé d'immersion ou de pulvérisation.
